# EUROPEAN PATENT SPECIFICATION

(11) **EP 2 214 732 B1**
(45) Date of publication and mention of the grant of the patent: **15.05.2013**
(21) Application number: 08838557.0
(22) Date of filing: 09.10.2008
(51) Int. Cl.: A61L 27/06, A61L 27/30, A61L 27/54

(54) **MULTIFUNCTIONAL TITANIUM SURFACES FOR BONE INTEGRATION**
MULTIFUNKTIONALE TITANOBERFLÄCHEN ZUR KNOCHENINTEGRATION
SURFACES DE TITANE MULTIFONCTIONNELLES POUR UNE INTÉGRATION DANS L'OS

(30) Priority: 12.10.2007 IT TO20070719
(43) Date of publication of application: 11.08.2010
(73) Proprietor: Politecnico di Torino, 10129 Torino (IT)
(72) Inventor: SPRIANO, Silvia, I-10126 Torino (IT); VERNE', Enrica, I-10138 Torino (IT); FERRARIS, Sara, I-10095 Grugliasco (Torino) (IT)
(74) Representative: Rambelli, Paolo
(86) International application number: PCT/IB2008/054148
(87) International publication number: WO 2009/047730

(56) References cited:
- EP-A1- 0 806 211
- WO-A2-2006/133027
- GB-A- 2 401 074
- DATABASE WPI Week 200360 Thomson Scientific, London, GB; AN 2003-633021 XP002562724 "Method for developing a bioactive implant of titanium and titanium alloy involves surface treatment" & KR 2003 0038631 A (LEE MIN HO [KR]) 16 May 2003 (2003-05-16)
- Gert Boere: "Influence of Fluoride on Titanium in an Acidic Environment Measured by Polarization Resistance Technique", Journal of Applied Biomaterials, 1 January 1995 (1995-01-01), pages 283-288, XP55023252, Retrieved from the Internet: URL:http://onlinelibrary.wiley.com/store/1 0.1002/jab.770060409/asset/770060409_ftp.p df?v=1&t=h0cg1deo&s=ed78b2869912a394ccb4e7 20b6f6734408c67f7c [retrieved on 2012-03-28]

## Description

This invention is referred to a process for the preparation of prosthetic devices that include titanium or titanium alloys, generally implantable devices constituted all or in part of titanium and/or its alloys, through a surface modification treatment to produce a bioactive surface.

The improvement of bone implant integration and tissue regeneration at the damaged site is still an open problem in orthopedic and dental surgery. In this way it is interesting to provide implants with multifunctional biomimetic surfaces capable of stimulating tissue regeneration both from an inorganic and from a biological point of view.

Within this frame the invention provides for surface modification of titanium or its alloys (used for orthopedic prostheses, dental implants or generally medical implants made with titanium or its alloys) through a thermochemical treatment and optionally surface functionalization with biomolecules, in order to get in vivo a bioactive behavior both from inorganic and biological point of view.

The term inorganic bioactivity indicates the ability of the surface to adsorb particularly Ca and P from physiological fluids and to induce hydroxyapatite (mineral constituent of bone) precipitation. On the other hand biological bioactivity means the ability of the surface to stimulate biological response (cellular adhesion, proliferation and differentiation) in order to quicken the growth of organic fraction of bone.

A surface able to stimulate simultaneously both activities, with a synergistic interaction between them, is a multifunctional material suited for rapid and effective bone integration of a permanent implant.

This behavior for titanium and its alloys is due to the chemical composition, crystallographic structure, morphology and surface energy of the surface oxide layer which is present on the metal.

Several treatments have been proposed, in scientific literature and patents, in order to obtain inorganic bioactivity of titanium and its alloys.

US 5 609 633 describes a material for osseous implants which includes a titanium substrate and a surface layer constituted of titanium oxide and an amorphous alkaline titanate, obtained by soaking titanium substrate into an alkaline solution and then thermally treating the material in order to form a stabilized alkaline titanate layer.

US 6 183 255 describes dental or orthopedic implants, with biological functionality, obtained through a chemical treatment in sodium hydroxide or through an acid treatment followed by an alkaline treatment and optionally by air oxidation.

In literature the use of hydrogen hydroxide is also reported to induce bioactivity, as described in Biomaterials, 23, 1353-1357 (2002), Biomaterials, 23, 3103-3111 (2002), Biomaterials, 22, 875 (2001) e Surface & Coatings Technology, 195, 252-257 (2005).

KR 20030038631 discloses a titanium or a titanium alloy implant with a bioactive surface made through a process comprising an acid attack with a mixture of hydrofluoric acid and nitric acid and a surface oxidation with a solution of hydrogen peroxide and metal chlorides.

EP 0 806 211 A1 describes a two-step surface treatment for a titanium implants with the use of a strong acid solution, such as hydrochloric, sulphuric, perchloric acids or a combination thereof, optionally followed by oxidising agents such as a nitric acid, peracids, hydroperoxides or hydrogen peroxide, optionally followed by neutralising steps.

The aim of the present invention is to provide a process to modify titanium or titanium alloys surfaces through a thermo chemical treatment in order to obtain an highly hydroxylated surface. This method is favourable because it does not require long time treatments in aggressive solutions, it does not mechanically damage the surfaces and does not compromise fatigue resistance.

A second aim of the invention is to provide a process to prepare a surface particularly suitable for a subsequent optional treatment step wherein the surface is functionalized with biomolecules.

Considering the described aims, the object of the present invention is a process as defined by the following claims.

In the annexed drawings:
- Figure 1 is a SEM micrography that shows surface microroughness of titanium surface after acid attack (a) and after both acid attack and hydrogen peroxide treatment (b);
- Figure 2 is an XPS spectrum of oxygen region, it shows hydroxiles exposition for native oxide (a) and for the modified surface (b);
- Figure 3 is a SEM micrography that shows hydroxyapatite precipitation onto material surface modified according to the invention after 15 days soaking in SBF;
- Figure 4 is an XRD spectrum after 15 days soaking in SBF;
- Figure 5 shows XRD spectra for samples after chemical and thermal treatment;
- Figure 6 shows results of XPS analysis for samples activated by means of trifluoroethanesulfonyl chloride;
- Figure 7 shows XPS spectra for samples functionalised with alkaline phosphatase (ALP): Survay analysis (a), carbon region detail (b); and
- Figure 8 is an histogram that shows enzymatic activity for functionalised samples.

### Inorganic bioactivity

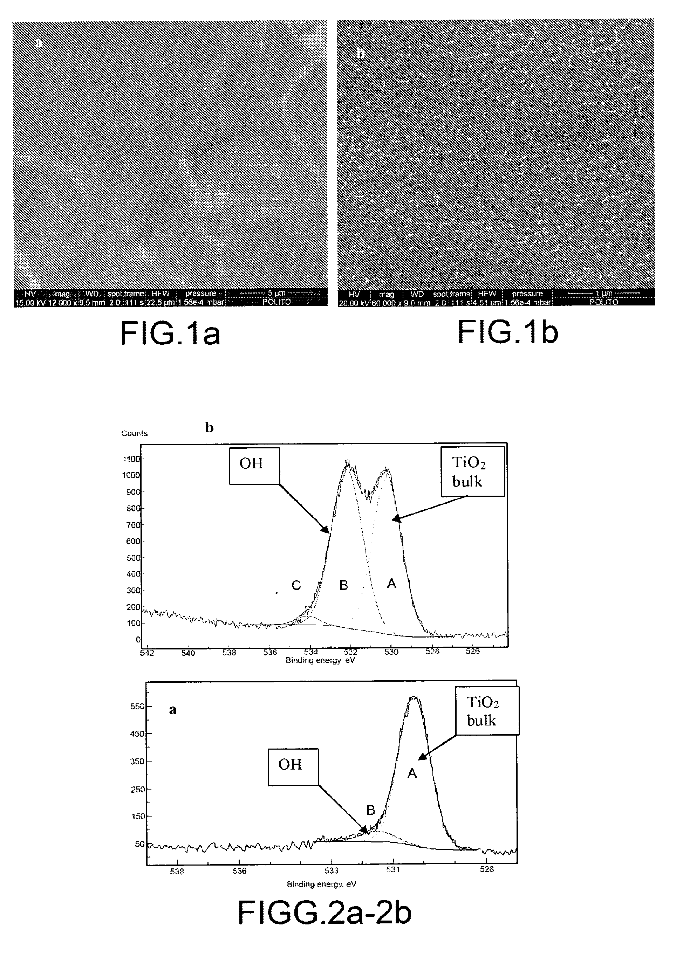

The first step of the treatment is an acid attack in order to remove native oxide present onto titanium (or titanium alloy) surface and make it macrorough. Acid attack is carried out using diluted hydrofluoric acid, for example with a concentration from 3M to 8M, for a time comprised between 1 and five minutes. Figure 1a shows macrorough titanium surface treated with 5 M hydrofluoric acid for 3 minutes.

This treatment is followed by a controlled oxidation , performed with a solution of hydrogen peroxide with a concentration in the range 15-70 volumes, preferentially at a temperature between 20°C and 80°C for a time in the range 30 - 400 minutes, best if between 30 and 225 minutes.

Figure 1b shows surface microroughness after 40 volumes hydrogen peroxide oxidation for 225 minutes at 60°C.

The oxidation process must be carried out so as to avoid surface re-passivation between the two passages (acid attack and controlled oxidation).

Process parameters have been optimized in order to avoid surface cracks formation. The obtained oxide layer presents an high number of hydroxyl groups (fig 2b) and a typical morphology characterized by roughness and porosity in the sub-micrometric scale (fig. 1b). Hydroxyl groups have a binding energy comprised in the range 531,5 - 532,2 eV, which is intermediate between acid OH groups and basic ones, as reported in J. Biomed. Mater. Res. 55, 185-193 (2001).

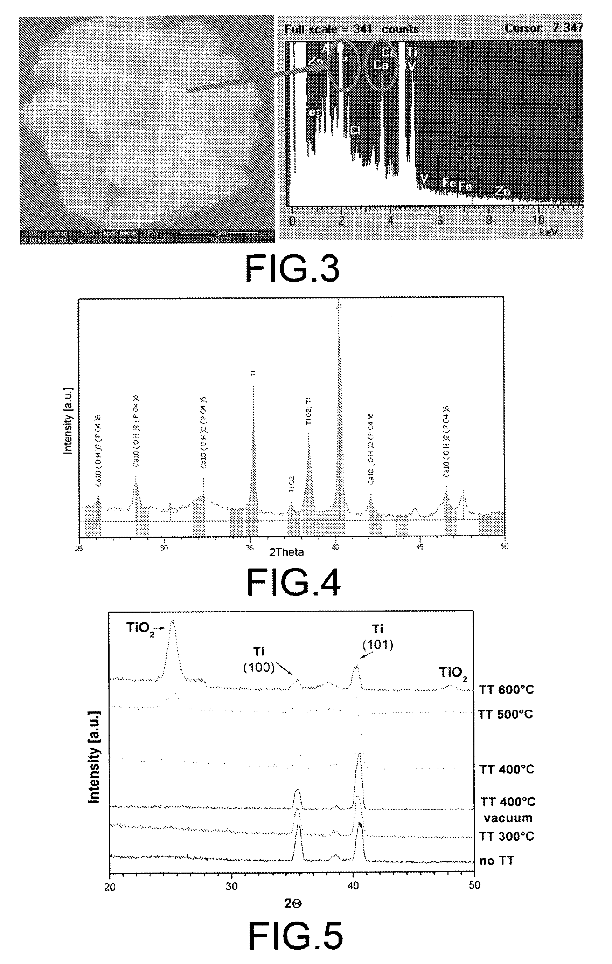

So treated material is bioactive from the inorganic point of view. In fact it promotes hydroxyapatite precipitation after 15 days soaking in simulated body fluid (SBF). SEM micrography (fig 3) shows the typical morphology of precipitates, that are aggregates of small spherical particles. EDS spectrum underlines their qualitative chemical composition. Ca/P rate is 1,7, so very close to the stoichiometric one for hydroxyapatite (1,67). Sample surface part which is not covered by particles presents an enrichment in calcium and phosphorous after soaking in SBF. This means a general ability of the surface to adsorb these elements. XRD analysis (fig 4) confirms hydroxyapatite presence onto the material surface; it is present in a partially amorphous phase with crystallites of small dimensions, as can be deduced from the presence of broad diffraction peaks.

Chemically treated samples (first with HF and then with H₂O₂) have been then thermally treated in order to stabilize the oxide layer. Thermal treatment could be performed in a temperature range between 300°c and 600°C, in air, in vacuum or in inert atmosphere, for a time comprised between 1 and 8 hours. Morphology and bioactivity are maintained after the thermal treatment.

Hydroxyl groups are stable to temperature, in fact their presence have been detected after prolonged storage and thermal treatment.

The thickness and the crystallinity of the oxide layer could be tailored varying temperature and atmosphere of the thermal treatment (fig 5). A progressive increment of the reflection peaks of anatase with increasing the temperature of the treatment can be observed, as expected.

Fatigue tests underline a low decrement in tensile strength (2-15%) after the treatment; so this process does not significantly alter the mechanical properties of the material.

Biological tests have been performed on human osteoblast like cells (MG63) in order to evaluate material-cells interaction. No difference has been observed in LDH production between natural titanium, modified titanium and control: this is an index that the treatment does not induce any significant cytotoxic effect.

It has been observed that cells adhere both on natural titanium and on treated one with the typical polygonal morphology, but it could be underlined that on modified surfaces cells appear more spread, and present more dorsal ruffles and numerous elongated filopodies than on Ti reference.

Osteoblasts cultured on superficially modified titanium surface show a good proliferation rate and a production of ALP, collagen I and osteocalcin significantly higher than on control samples. This means that osteoblasts cultured onto the modified surface have an higher degree of differentitation if compared to untreated titanium and control. So the simple inorganic modification influences the biological response of the material.

### Biological bioactivity

Preferably, the process considered in the invention includes - as further stage - a treatment of surface functionalization of titanium or its alloys with organic molecules (natural or synthetical) involved in bone mineralization (such as protein from the extracellular matrix, peptides, or enzymes involved in adhesion/proliferation processes, bone morphogenetic proteins, growth factors, albumin, fibronectin, alkaline phosphatase and similar). Surface functionalization is advantageous because it allows the grafting of a controlled amount of the biomolecules, avoiding an excessive use such as resulting from local injection or systemic delivery.

Prosthetic devices or medical implants with titanium surfaces functionalized with biomolecules are described in US 2007/077 346 and US 2004/083 006. In US 2007/077 346 titanium surfaces are coated with the corresponding hydride that contains one or more biomolecules; the functionalization process is carried out through an electrolytic treatment in presence of the biomolecules.

In US 2004/083 006 metallic surfaces are coated with an hydroxide layer through an electrolysis process in presence of one or more biomolecules.

As far as this invention is concerned biomolecules able to induce cellular response are preferably covalently fixed onto titanium (or titanium alloy) surface previously made bioactive from the inorganic point of view. Direct covalent bonding has been chosen as grafting mechanism because it is more selective and allows a better adhesion than simple adsorption or release from a reabsorbable carrier.

The selected functionalization process avoid silanization and employment of other spacer molecules that could be toxic (such as glutaraldehyde). In particular for the described functionalisation process it is fundamental the exposition of hydroxyl groups onto the treated surface because they represent the reactive functional group useful for biomolecule grafting on the surface.

In particular, for this surface modification, acid attack and controlled oxidation have been carried out on the titanium surface as described in the previous section, subsequently said surface has been activated with an organic chloride, particularly with trifluoroethanesulfonyl chloride (TC), as already reported in literature; see for example "Biochemical Surface Modification of CO-Cr-MO", D. A. Puleo, Biomaterials (17) (1996), 217-222 e "Direct Attachment of Fibronectine to Tresylchloride Activated Titanium", T. Hayakawa et al., J. Biomed. Mater. Res. 67 (2003), 684-688.

In a particular example, biological functionalization has been carried out using the enzyme alkaline phosphatase (ALP), which is involved in mineralization processes of hard tissues; several literature works suggest that its local application promote growth and mineralization of damaged bone. Besides, ALP is a good model molecule because it is quite simple to detect. Nevertheless the biological functionalization step is not limited to the specific example described but it is impossible to employ generally proteins from extracellular matrix or enzymes involved in adhesion/proliferation processes, as said before.

Preferably, surface activation with trifluoroethanesulfonyl chloride is performed at a reaction temperature comprised between 20°C and 50°C for times from 10 minutes to 72 hours. Preferred conditions are 37°C for 48 hours.

The presence of an high number of hydroxyl groups on the surface is crucial for material response at this second phase of the treatment.

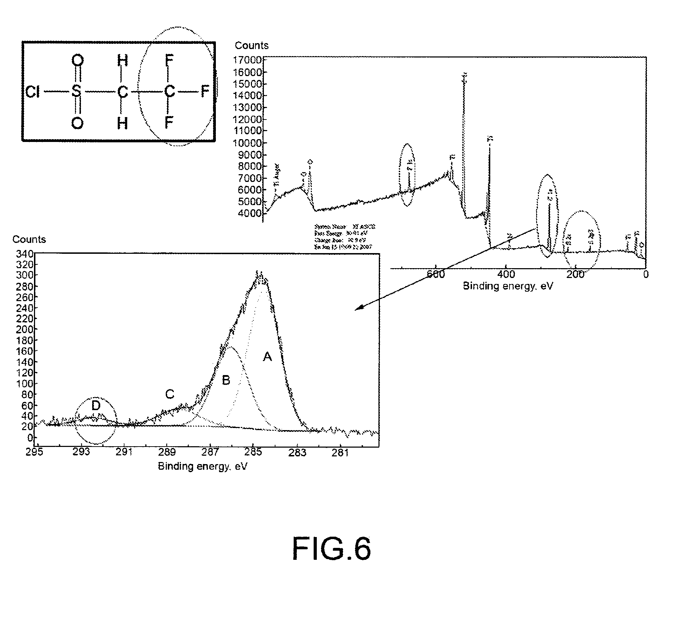

Trifluoroethanesulfonyl chloride presence onto material surface has been determined by means of XPS analysis (fig 6): D peak (at about 292 eV) is characteristic of C-F bonds.

The final step of the process is the enzymatic grafting to modified surfaces. For this purpose, a solution of the chosen biomolecule has to be prepared, diluting the biomolecule preferably in PBS, to reach a final concentration comprised between 2 and 40 mg/ml, soaking the samples for a time in the range 0.5 - 72 hours, at a temperature between 0°C and 50°C.

In a particular application of the process, a 5 mg/ml solution of ALP in PBS with an incubation of 24 hours at 4°C have been employed.

The result of the modification process is a direct anchoring of the molecule to the titanium surface, because trifluoroethanesulfonyl chloride is a good leaving group.

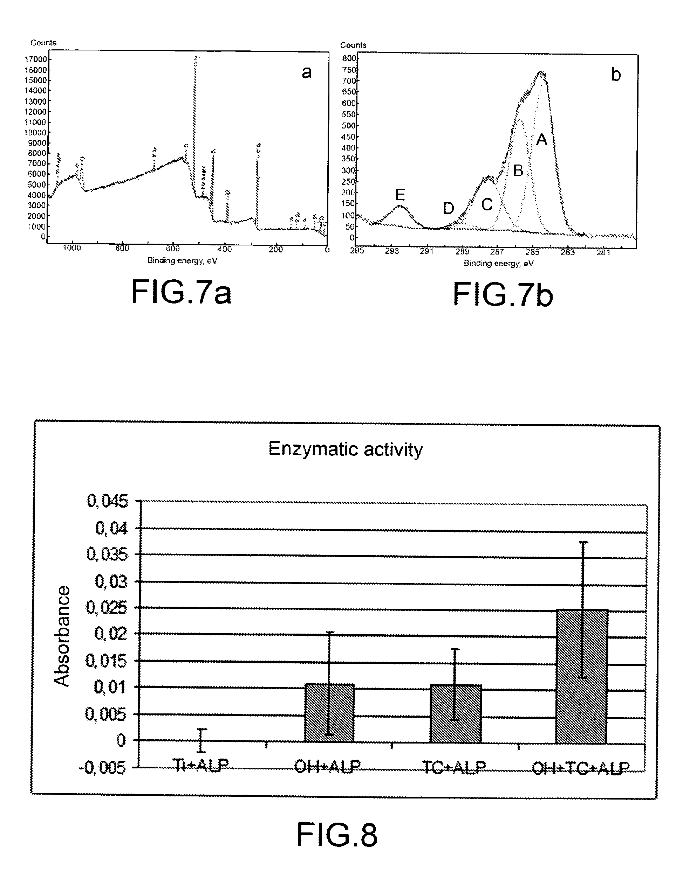

Enzyme presence has been determined by means of XPS analysis that shows an increment in carbon and nitrogen and a decrement in titanium oxide (fig. 7) on the outer layer of the modified surface. This is an index of an organic coating. The detailed study of carbon region underlines that the higher signal is due to C-O and C-N bonds, characteristic of the enzyme (fig 7 - peak B, at 286 eV). D peak (289 eV) could be attributed to aromatic rings of the enzyme flattened onto the surface. Both signals are significantly different from organic contamination always present onto titanium surfaces.

Enzymatic activity after anchoring on the surface has been determined by means of UV-visible analysis, after reaction with paranitrophenylphosphate. This reaction produces paranitrophenol, which has a yellow colouring in alkaline environment. Yellow intensity, spettrophotometrically evaluated, could be correlated to the ALP amount present onto material surface.

Results (fig 8) show that the enzyme anchored to the surface maintains its activity and that the entire functionalization process (hydroxylation, surface activation and molecular grafting) is required in order to obtain an effective functionalization.

## Claims

1. A process for the preparation of a prosthetic device or medical implant comprising titanium or titanium alloys, through a surface modification treatment, in order to make bioactive titanium or titanium alloy surface, comprising the following steps:
a) an acid attack with an acid solution consisting of diluted hydrofluoric acid thereby to remove native oxide present on said surface of said prosthetic device or medical implant; and
b) surface oxidation with an hydrogen peroxide solution, in order to produce hydroxyl groups onto the surface.

2. A process according to claim 1, wherein step b) is followed by step: c) thermal treatment in order to stabilize the oxide layer generated in step b).

3. A process according to claim 2, wherein said step c) is carried out at a temperature comprised between 300°C and 600°C, for times from 1 to 8 hours in air, vacuum or inert atmosphere.

4. A process according to any of claims 1 to 3 , wherein the acid attack of step a) is carried out in diluted hydrofluoric acid, with a molar concentration between 1 and 10, for a time in the range 0.5 - 30 minutes.

5. A process according to any of the previous claims, wherein surface oxidation of step b) is carried out in hydrogen peroxide with a concentration between 15 and 70 volumes at a temperature between 20°C and 80°C for a time in the range 30 - 400 minutes.

6. A process according to any of the previous claims, that includes, after step b) or c), a functionalization step of the titanium or titanium alloy surface with biomolecules **characterized by** biological bioactivity.

7. A process according to claim 6, wherein the functionalization treatment is performed by:
d) activation of the titanium or titanium alloy surface, previously treated according to steps a), b) and optionally c), with an organic chloride; e) incubation of the activated surfaces into a solution of the chosen biomolecule.

8. A process according to claim 7, wherein the activation step d) is carried out with trifluoroethanesulfonyl chloride at a reaction temperature between 20°C and 50°C, for a time included in the range from 10 minutes to 72 hours.

9. A process according to claims 7 or 8, wherein incubation step e) is carried out by soaking the activated surface in a solution with a concentration in the range between 2 and 40 mg/ml, for a time from 0.5 to 72 hours, at a temperature from 0°C to 50°C.

10. A process according to any of claims 6 to 9, wherein said biomolecule has been chosen among organic molecules, natural or synthetic, involved in bone mineralization processes.

11. A process according to any of claims 6 to 9, wherein said biomolecule is a biomolecule natural, recombinant or synthetic, chosen among bio-adhesives, cellular adhesion factors, biopolymers, blood proteins, enzymes, proteins or biomolecules from extracellular matrix, growth factors, hormones, peptides, deoxyribonucleic acid (DNA), ribonucleic acid (RNA), receptors, enzymatic inhibitors, drugs, anions and cations biologically active, peptides, morphogenetic proteins, adenosine monophosphate (AMP), adenosine diphosphate (ADP), adenosine triphosphate (ATP), albumin, fibronectin, alkaline phosphatase and combinations of the previous cited molecules.

12. Implantable device, particularly arthroprosthesis or dental implant totally or partially constituted of titanium or titanium alloys, with a titanium or titanium alloy surface made bioactive through a process according to any claim from 1 to 11 with a bioactive surface with bound hydroxyl groups, the binding energy of said hydroxyl groups being in the range 531,5 - 532,2 eV.

13. Implantable device according to claim 12 , with a titanium or titanium alloy surface lacking of surface cracks that presents micrometric roughness in combination with sub-micrometric roughness-porosity.

14. Implantable device according to claim 12, wherein the titanium or titanium alloy surface is functionalized with biomolecules covalently grafted to the surface.

15. Implantable device according to any of claims 12 to 14 in sterile form.

## Patentansprüche

1. Verfahren für die Herstellung einer prosthetischen Vorrichtung oder eines medizinischen Implantats, umfassend Titan oder Titanlegierungen, mit Hilfe einer Oberflächenmodifizierungsbehandlung, um eine bioaktive Titan- oder Titanlegierungsoberfläche zu erzeugen, umfassend die folgenden Schritte:
a) Einen Säureangriff mit einer sauren Lösung, bestehend aus verdünnter Flußsäure, um dadurch natives Oxid, das auf der Oberfläche der prosthetischen Vorrichtung oder des medizinischen Implantats vorhanden ist, zu entfernen und
b) Oberflächenoxidation mit einer Wasserstoffperoxidlösung, um Hydroxylgruppen auf der Oberfläche zu erzeugen.

2. Verfahren nach Anspruch 1, wobei Schritt b) gefolgt wird vom Schritt:
c) Wärmebehandlung, um die in Schritt b) erzeugte Oxidschicht zu stabilisieren.

3. Verfahren nach Anspruch 2, wobei Schritt c) bei einer Temperatur zwischen 300°C, und 600°C für eine Dauer von 1 bis 8 Stunden in Luft, Vakuum oder einer inerten Atmosphäre ausgeführt wird.

4. Verfahren nach einem der Ansprüche 1 bis 3, wobei der Säureangriff von Schritt a) in verdünnter Flußsäure mit einer molaren Konzentration zwischen 1 und 10 für eine Dauer im Bereich von 0,5 bis 30 Minuten durchgeführt wird.

5. Verfahren nach einem der vorhergehenden Ansprüche, wobei die Oberflächenoxidation von Schritt b) in Wasserstoffperoxid mit einer Konzentration zwischen 15 und 70 Volumina bei einer Temperatur zwischen 20°C und 80°C für eine Dauer im Bereich von 30 bis 400 Minuten durchgeführt wird.

6. Verfahren nach einem der vorhergehenden Ansprüche, das, nach Schritt b) oder c), einen Funktionalisierungsschritt der Titan- oder Titanlegierungsoberfläche mit Biomolekülen, die durch biologische Bioaktivität gekennzeichnet sind, enthält.

7. Verfahren nach Anspruch 6, wobei die Funktionalisierungsbehandlung durchgeführt wird durch:
d) Aktivierung der Titan- oder Titanlegierungsoberfläche, die vorher entsprechend den Schritten a), b) und gegebenenfalls c) behandelt wurde, mit einem organischen Chlorid,
e) Inkubation der aktivierten Oberflächen in einer Lösung des gewählten Biomoleküls.

8. Verfahren nach Anspruch 7, wobei der Aktivierungsschritt d) mit Trifluorethansulfonylchlorid bei einer Reaktionstemperatur zwischen 20°C und 50 °C, für eine Dauer im Bereich von 10 Minuten bis 72 Stunden durchgeführt wird.

9. Verfahren nach Anspruch 7 oder 8, wobei der Inkubationsschritt e) durch Einweichen der aktivierten Oberfläche in einer Lösung mit einer Konzentration im Bereich zwischen 2 und 40 mg/ml für eine Dauer von 0,5 bis 72 Stunden bei einer Temperatur von 0°C bis 50°C durchgeführt wird.

10. Verfahren nach einem der Ansprüche 6 bis 9, wobei das Biomolekül aus organischen Molekülen, natürlich oder synthetisch, die an Knochenmineralisierungsprozessen beteiligt sind, ausgewählt wurde.

11. Verfahren nach einem der Ansprüche 6 bis 9, wobei das Biomolekül ein natürliches, rekombinantes oder synthetisches Biomolekül ist, ausgewählt aus Bioklebstoffen, zellulären Adhäsionsfaktoren, Biopolymeren, Blutproteinen, Enzymen, Proteinen oder Biomolekülen aus der extrazellulären Matrix, Wachstumsfaktoren, Hormonen, Peptiden, Deoxyribonukleinsäure (DNA), Ribonukleinsäure (RNA), Rezeptoren, enzymatischen Inhibitoren, Arzneistoffen, biologisch aktiven Anionen und Kationen, Peptiden, morphogenetischen Proteinen, Adenosinmonophosphat (AMP), Adenosindiphosphat (ADP), Adenosintriphosphat (ATP), Albumin, Fibronectin, alkalischer Phosphatase und Kombinationen der vorstehend genannten Moleküle.

12. Implantierbare Vorrichtung, insbesondere Arthroprothese oder dentales Implantat, das gesamt oder teilweise aus Titan oder Titanlegierungen besteht, mit einer Titan- oder Titanlegierungsoberfläche, die mit Hilfe eines Verfahrens nach einem der Ansprüche 1 bis 11 mit einer bioaktiven Oberfläche mit gebundenen Hydroxylgruppen bioaktiv gemacht wurde, wobei die Bindungsenergie der Hydroxylgruppen im Bereich von 531,5 bis 532,2 eV liegt.

13. Implantierbare Vorrichtung nach Anspruch 12, mit einer Titan- oder Titanlegierungsoberfläche, die keine Oberflächenrisse aufweist, welche mikrometrische Rauheit in Verbindung mit submikrometrischer Rauheit/Porosität bereitstellt.

14. Implantierbare Vorrichtung nach Anspruch 12, wobei die Titan- oder Titanlegierungsoberfläche mit Biomolekülen funktionalisiert ist, die kovalent auf die Oberfläche aufgepfropft wurden.

15. Implantierbare Vorrichtung nach einem der Ansprüche 12 bis 14 in steriler Form.

## Revendications

1. Procédé de préparation d'une prothèse ou d'un implant médical comprenant du titane ou des alliages de titane, par l'intermédiaire d'un traitement de modification de surface, afin de former une surface de titane ou d'alliage de titane bioactif, comprenant les étapes suivantes :
a) une attaque acide avec une solution acide constituée d'acide fluorhydrique dilué afin de supprimer l'oxyde natif présent sur ladite surface de ladite prothèse ou dudit implant médical ; et
b) une oxydation de surface avec une solution de peroxyde d'hydrogène, afin de produire des groupes hydroxyle sur la surface.

2. Procédé selon la revendication 1, dans lequel l'étape b) est suivie de l'étape :
c) un traitement thermique afin de stabiliser la couche d'oxyde générée à l'étape b).

3. Procédé selon la revendication 2, dans lequel ladite étape c) est exécutée à une température comprise entre 300 °C et 600 °C, avec des durées de 1 à 8 heures dans l'air, le vide ou en atmosphère inerte.

4. Procédé selon l'une quelconque des revendications 1 à 3, dans lequel l'attaque acide de l'étape a) est réalisée dans de l'acide fluorhydrique dilué, avec une concentration molaire de 1 à 10, pendant une durée comprise dans l'intervalle de 0,5 à 30 minutes.

5. Procédé selon l'une quelconque des revendications précédentes, dans lequel l'oxydation de surface de l'étape b) est réalisée dans du peroxyde d'hydrogène ayant une concentration de 15 à 70 volumes, à une température de 20 °C à 80 °C pendant une durée comprise dans l'intervalle de 30 à 400 minutes.

6. Procédé selon l'une quelconque des revendications précédentes, qui comprend, après l'étape b) ou c), une étape de fonctionnalisation de la surface en titane ou en alliage de titane avec des biomolécules **caractérisées par** une bioactivité biologique.

7. Procédé selon la revendication 6, dans lequel le traitement de fonctionnalisation est réalisé par :
d) une activation de la surface de titane ou d'alliage de titane, préalablement traitée selon les étapes a), b) et éventuellement c), avec un chlorure organique ;
e) une incubation des surfaces activées dans une solution de la biomolécule choisie.

8. Procédé selon la revendication 7, dans lequel l'étape d'activation d) est réalisée avec du chlorure de trifluoroéthanesulfonyle à une température de réaction de 20 °C à 50 °C, pendant une durée comprise dans l'intervalle de 10 minutes à 72 heures.

9. Procédé selon la revendication 7 ou 8, dans lequel l'étape d'incubation e) est réalisée en immergeant la surface activée dans une solution ayant une concentration de 2 à 40 mg/ml, pendant une durée de 0,5 à 72 heures, à une température de 0 °C à 50 °C.

10. Procédé selon l'une quelconque des revendications 6 à 9, dans lequel ladite biomolécule a été choisie parmi les molécules organiques, naturelles ou synthétiques, impliquées dans les processus de minéralisation des os.

11. Procédé selon l'une quelconque des revendications 6 à 9, dans lequel ladite biomolécule est une biomolécule naturelle, recombinée ou synthétique, choisie parmi les bio-adhésifs, les facteurs d'adhésion cellulaire, les biopolymères, les protéines du sang, les enzymes, les protéines ou les biomolécules provenant de la matrice extracellulaire, les facteurs de croissance, les hormones, les peptides, l'acide désoxyribonucléique (ADN), l'acide ribonucléique (ARN), les récepteurs, les inhibiteurs enzymatiques, les médicaments, les anions et les cations biologiquement actifs, les peptides, les protéines morphogénétiques, l'adénosine monophosphate (AMP), l'adénosine diphosphate (ADP), l'adénosine triphosphate (ATP), l'albumine, la fibronectine, la phosphatase alcaline et les combinaisons des molécules précédemment citées.

12. Dispositif implantable, en particulier arthroprothèse ou implant dentaire totalement ou partiellement constitué(e) de titane ou d'alliage de titane, avec une surface en titane ou en alliage de titane rendue bioactive au moyen d'un procédé selon l'une quelconque des revendications 1 à 11 avec une surface bioactive à groupes hydroxyle liés, l'énergie de liaison desdits groupes hydroxyle étant comprise dans l'intervalle de 531,5 à 532,2 eV.

13. Dispositif implantable selon la revendication 12, avec une surface en titane ou en alliage de titane exempte de craquelures en surface qui présente une rugosité micrométrique en combinaison avec une porosité de rugosité sous micrométrique.

14. Dispositif implantable selon la revendication 12, dans lequel la surface en titane ou en alliage de titane est fonctionnalisée avec des biomolécules greffées à la surface de façon covalente.

15. Dispositif implantable selon l'une quelconque des revendications 12 à 14 sous forme stérile.
